# EUROPÄISCHE PATENTSCHRIFT

(11) **EP 1 819 280 B1**
(45) Veröffentlichungstag und Bekanntmachung des Hinweises auf die Patenterteilung: **14.08.2013**
(21) Anmeldenummer: 05797446.1
(22) Anmeldetag: 18.10.2005
(51) Int. Cl.: A61B 17/02, A61B 17/17, A61B 17/15

(54) **BÄNDERSPANNVORRICHTUNG UND SCHNITTLEHRE ZUR OSTEOTOMIE**
LIGAMENT-TENSIONING DEVICE AND CUTTING JIG FOR OSTEOTOMY
DISPOSITIF DE TENSION DE LIGAMENTS ET GABARIT DE COUPE POUR PROCÉDÉ D'OSTEOTOMIE

(30) Priorität: 19.10.2004 DE 102004050913
(43) Veröffentlichungstag der Anmeldung: 22.08.2007
(73) Patentinhaber: Mathys AG Bettlach, 2544 Bettlach (CH)
(72) Erfinder: DELFOSSE, Daniel, CH-3303 Jegenstorf (CH); SUPPER, Walter, CH-2540 Grenchen (CH); FRANKHAUSER, Christoph, CH-4500 Solothurn (CH)
(74) Vertreter: Körfer, Thomas
(86) Internationale Anmeldenummer: PCT/EP2005/011203
(87) Internationale Veröffentlichungsnummer: WO 2006/042743

(56) Entgegenhaltungen:
- EP-A- 0 466 659
- EP-A- 0 809 969
- WO-A-00/78225
- WO-A-01/85038
- WO-A-03/084412
- US-A- 5 688 280
- US-A- 5 716 361
- US-A1- 2002 068 942
- RITSCHL P ET AL EUROPEAN ASSOCIATION FOR COMPUTER GRAPHICS: "COMPUTER ASSISTED LIGAMENT BALANCING IN TKR USING THE PIGALILEO SYSTEM" POSTER PRESENTATIONS. VIENNA, SEPT. 2 - 6, 1991, PROCEEDINGS OF THE EUROPEAN COMPUTER GRAPHICS CONFERENCE AND EXHIBITION, AMSTERDAM, NORTH HOLLAND, NL, Bd. CONF. 12, 18. Juni 2003 (2003-06-18), XP009052311

## Beschreibung

Die Erfindung betrifft eine Bänderspannvorrichtung für Gelenke des menschlichen oder tierischen Körpers. Offenbart ist auch eine zur Verwendung an einem mittels der Bänderspannvorrichtung vorbereiteten Gelenks geeignete Schnittlehre sowie ein Verfahren zur Osteotomie dieser Gelenke unter Verwendung der erfindungsgemäßen Bänderspannvorrichtung und der Schnittlehre.

Aus der WO 03/084412 A1 ist eine Bänderspannvorrichtung zur Vorbereitung für die Implantierung eines Gelenksimplantats mit einem Grundkörper bekannt, welcher eine erste Pratze mit einer distalen Anlagefläche, welche auf einem ersten Knochen aufliegt, und eine zweite Pratze, die mit einer proximalen Auflagefläche an einem zweiten Knochen anliegt, aufweist. Die zweite Pratze ist parallel zur ersten Pratze verschiebbar. Eine Schnittlehre ist auf Halterungen des Grundkörpers der Bänderspannvorrichtung aufsetzbar.

Auch aus der WO 00/78225 A1 ist eine Bänderspannvorrichtung für nicht-kugelige Gelenke bekannt. Die darin beschriebene Vorrichtung zum Spannen von Bändern an nicht-kugeligen Gelenken am menschlichen oder tierischen Körper umfaßt einen prismatischen, zylindrischen oder plattenförmigen Grundkörper mit einer rechten Pratze und einer linken Pratze, welche erste Auflageflächen in einer Ebene aufweisen und damit parallel auf die gelenkseitige Oberfläche eines ersten an ein nicht-kugeliges Gelenk angrenzenden Knochens zur Anlage bringbar sind, sowie einen rechten Handgriff und einen linken Handgriff, einen rechten Spannhebel und einen linken Spannhebel mit zweiten Auflageflächen, welche parallel zu den ersten Auflageflächen angeordnet sind, wobei zwischen den jeweiligen Auflageflächen des rechten Spannhebels und der rechten Pratze eine Spannweite Y und zwischen den jeweiligen Auflageflächen des linken Spannhebels und der linken Pratze dieselbe oder eine andere Spannweite X einstellbar ist. Die zweiten Auflagefläche sind auf die gelenkseitige Oberfläche eines zweiten an das Gelenk angrenzenden Knochens zur Anlage bringbar. Weiterhin umfaßt die Vorrichtung einen rechten Bedienungshebel und einen linken Bedienungshebel, welche gleichzeitig mit dem Halten der Vorrichtung mit je einer Hand am entsprechenden Handgriff einzeln mit der jeweils selben Hand betätigbar sind und eine rechte Parallelverschiebevorrichtung und eine linke Parallelverschiebevorrichtung, welche je durch den entsprechenden Bedienungshebel antreibbar sind und so mit je einem Spannhebel verbunden sind, daß bei einer Bewegung der Bedienungshebel die Spannweiten X bzw. Y unabhängig voneinander einstellbar sind. Die Parallelverschiebevorrichtungen sind als Viergelenk-Hebelgetriebe ausgebildet.

Nachteilig an den aus den oben genannten Druckschriften bekannten Bänderspannvorrichtungen ist insbesondere, daß die Anbringung von Schnittebenen an einem erkrankten Gelenk zur Einbringung einer Prothese weitere Werkzeuge erfordert, welche unabhängig von der Spannvorrichtung an das Gelenk angesetzt werden und dadurch keine genaue Positionierung und Ausrichtung sowie keine reproduzierbare, genaue Schnittführung erlauben.

Der Erfindung liegt demnach die Aufgabe zugrunde, eine Bänderspannvorrichtung zu schaffen, um die Kapsel-Bandstrukturen eines prothetisch zu versorgenden Gelenkes mit einer parallelen Spreizbewegung anzuspannen und dabei eine voreinstellbare, nachjustierbare und nachkontrollierbare Resektionshöhe für die mediale und die laterale Seite getrennt voneinander einstellbar sind.

Die Aufgabe wird hinsichtlich der Bänderspannvorrichtung durch die Merkmale des Anspruchs 1 gelöst.

Weitere vorteilhafte Ausgestaltungen der Erfindung sind in den Unteransprüchen gekennzeichnet.

Die Erfindung wird im folgenden anhand teilweise schematischer Darstellungen für die Vorbereitung der prothetischen Versorgung eines menschlichen Kniegelenks näher erläutert.

In der Zeichnung zeigen:
- Fig. 1A: eine schematische, perspektivische Ansicht einer erfindungsgemäßen Bänderspannvorrichtung mit einer Bohrlehre,
- Fig. 1B: eine vergrößerte Darstellung der in Fig. 1A dargestellten Bänderspannvorrichtung in einer Ansicht mit Blickrichtung nach dorsal,
- Fig. 1C: eine schematische, perspektivische Ansicht einer erfindungsgemäßen Bänderspannvorrichtung mit Blickrichtung nach medial,
- Fig. 2A-C: schematische, perspektivische Darstellungen einer distalen Femurosteotomie unter Verwendung einer Schnittlehre,
- Fig. 3A-D: schematische, perspektivische Darstellungen des Ansatzes einer Bohrlehre zur Vorbereitung der Bohrungen für die Schnittlehre,
- Fig. 4A-C: schematische, perspektivische Darstellungen des Ansatzes einer Tastlehre zur Ermittlung der Femurgröße,
- Fig. 5: eine schematische, perspektivische Darstellung des zum Ansatz der Schnittlehre vorbereiteten Kniegelenks, und
- Fig. 6A-D: schematische, perspektivische Darstellungen der Schnittlehre ex situ und in situ.

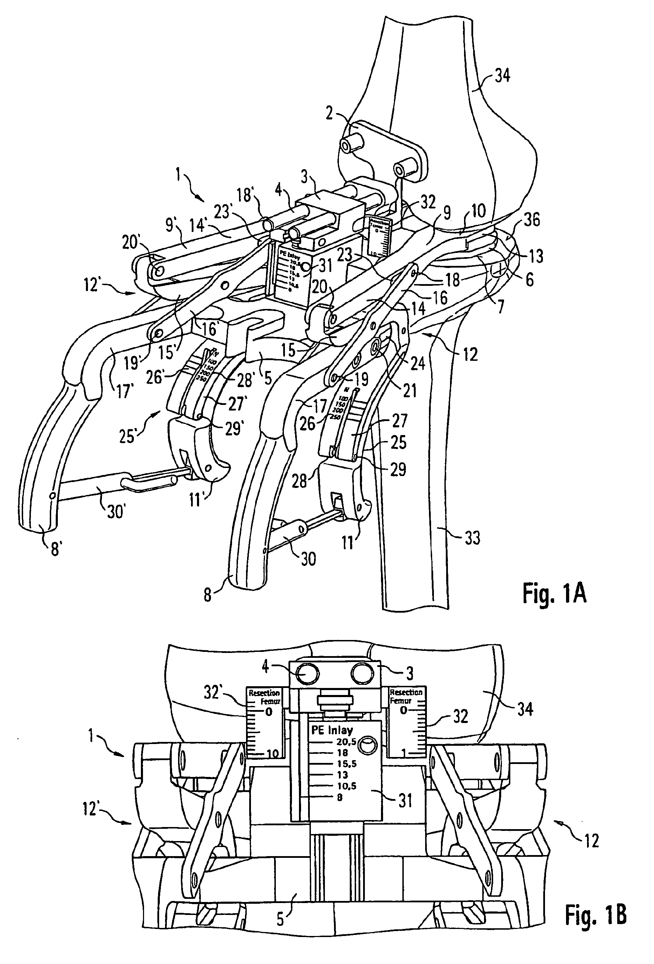

Fig. 1A zeigt in einer schematischen, perspektivischen Gesamtdarstellung eine Bänderspannvorrichtung 1, die einen Grundkörper 5 umfaßt, auf welchem ein Führungskörper 3 mit Führungsstäben 4 angeordnet ist. Auf die Führungsstäbe 4 sind verschiedene Bohrlehren 2 zur Vorbereitung von Resektionen im Bereich eines prothetisch zu versorgenden Gelenks, beispielsweise eines Kniegelenks, aufsteckbar und verschiebbar.

Die Bänderspannvorrichtung 1 umfaßt zur sicheren Einleitung der Spreizkraft in ein erstes Knochenteil 33 erste Pratzen 6, 6' (in Fig. 1A nicht sichtbar) mit ersten Auflageflächen 7, 7' (in Fig. 1A ebenfalls nicht sichtbar), welche im Fall des Kniegelenks auf der Tibia 33 (Schienbeinkopf) aufliegen. Den ersten Pratzen 6, 6' gegenüberliegend sind entsprechend am Grundkörper 5 Handgriffe 8, 8' angebracht, welche jeweils ein einhändiges Halten und Spannen der Bänderspannvorrichtung 1 ermöglichen. Ebenfalls entsprechend zur Anordnung der ersten Pratzen 6, 6' und oberhalb dieser liegend umfaßt die Bänderspannvorrichtung 1 Spannhebel 9, 9', welche sich mit ihrer auf zweiten Pratzen 13, 13' (in Fig. 1A ebenfalls nicht sichtbar) ausgebildeten zweiten Auflageflächen 10, 10' auf einem zweiten, gegenüberliegenden Knochenteil 34 des zu behandelnden Gelenkes, im Fall des Kniegelenks dem Femur 34, abstützen. Die Spreizwirkung wird durch Betätigen der Handgriffe 8, 8' zusammen mit jeweils einem Bedienungshebel 11, 11' für einen medialen oder lateralen Gelenkanteil getrennt oder gemeinsam erzeugt. Parallelverschiebevorrichtungen 12, 12' gestatten bezüglich der Auflageflächen 7, 7' und 10, 10' eine Parallelverschiebung der zweiten Pratzen 13, 13' mit den Auflageflächen 10, 10' gegenüber den ersten Pratzen 6, 6' mit den Auflageflächen 7, 7'. Die zweiten Pratzen 13, 13' stehen dabei in Wirkverbindung mit den Spannhebeln 9, 9'.

Die Parallelverschiebevorrichtungen 12, 12' sind als Viergelenk in Form sich kreuzender Stäbe ausgeführt und umfassen jeweils vier Hebel 14, 14', 15, 15', 16, 16', 17, 17', wobei spannhebelseitiger Hebel 14, 14' und grundkörperseitige Hebel 17, 17' parallel zueinander angeordnet sind, während sich die Hebel 15 und 16 bzw. 15' und 16' kreuzen. Die jeweils vier Hebel 14, 15, 16, 17 bzw. 14', 15', 16', 17' sind mittels jeweils fünf Achsen 18, 19, 20, 21, 22 bzw. 18', 19', 20', 21', 22' miteinander verbunden. Zwei der Achsen 18 , 19 bzw. 18' 19' sind in den parallelen Hebeln 14, 17 bzw. 14', 17' in parallel zu den Auflageflächen 7, 7', 10, 10' verlaufenden Langlöchern 23, 23', 24, 24' verschiebbar gelagert. Diese Ausgestaltung der Parallelverschiebevorrichtungen 12, 12' gestattet, daß die spannhebelseitigen Hebel 14, 14' und die grundkörperseitigen Hebel 17, 17' parallel zueinander bzw. auseinander bewegbar sind.

Die Längen der Hebel 14, 14' 15, 15', 16, 16', 17, 17' sind so gewählt, daß bei einer beliebigen Spannweite X zwischen den Auflageflächen 7, 7' an den ersten Pratzen 6, 6' und den Auflageflächen 10, 10' an den zweiten Pratzen 13, 13', welche z.B. zwischen 5 mm und 40 mm liegen kann, ein konstantes Umsetzungsverhältnis von 1:1 zwischen der manuell an den Handgriffen 8, 8' und den Bedienungshebeln 11, 11' aufgebrachten Spannkraft und der auf die an das Gelenk angrenzenden Knochen ausgeübten Distraktionskraft herrscht.

Die Größe der Spreizkraft ist an Kraftanzeigen 25, 25' mit Skalen 26, 26' und beweglichen Anzeigehebeln 27, 27' ablesbar. Die Anzeigehebel 27, 27' werden durch die longitudinale Biegung der durch eine manuell aufgebrachte Spannkraft biegbaren Bedienungshebelteile 28, 28' gegenüber den anderen gabelartig angeordneten und nicht durch diese Spannkraft beaufschlagten Anzeigehebeln 27, 27' bewegt. Werden mittels der Spannkraft der Anzeigehebel 27, 27' und die Bedienungshebelteile 28, 28' relativ zueinander bewegt, drehen sich die Anzeigehebel 27, 27' um Drehachsen 29, 29', wodurch auf den Skalen 26, 26' durch die Anzeigehebel 27, 27' die manuell aufgebrachte Spannkraft angezeigt wird.

Weiterhin können zwischen den Handgriffen 8, 8' und den Bedienungshebeln 11, 11' Arretierungsvorrichtungen 30, 30' vorgesehen sein, welche die Arretierung der Bänderspannvorrichtung 1 in einer bestimmten Position ermöglichen.

Der Grundkörper 5 der Bänderspannvorrichtung 1 weist eine erste Skala 31 auf, welche mit zweiten Skalen 32, 32' korrespondiert. Die Skalen 31, 32 und 32' (in Fig. 1A ebenfalls nicht sichtbar) zeigen die geplante Resektionshöhe medial und lateral am Knochen, z.B. am Femur 34 unter Berücksichtigung der ligamentären Situation und bei bereits resezierter Tibia 33 an, wodurch vor der Resektion dorsal und ventral die Resektionshöhen medial und lateral gemessen werden können. Durch die Wahl der femoralen Resektionshöhe ist eine optimale Reproduktion der physiologischen Gelenksebene möglich. Die genaue Funktion der Bänderspannvorrichtung 1 ist in den folgenden Figuren sowie in der dazugehörigen Beschreibung näher erläutert.

Hierbei wurde in den Figuren auf eine Wiederholung der Bezugszeichen für die nicht für die Erfindung relevanten Bauteile aus Gründen der Übersichtlichkeit verzichtet. Nur einige, für die Orientierung hilfreichen Teile sind bezeichnet. Ebenso wurde auf eine wiederholende Beschreibung der entsprechenden Bauteile in der nachfolgenden Beschreibung verzichtet.

Fig. 1B zeigt in einer schematischen Ansicht mit Blickrichtung nach dorsal eine Aufsicht auf die Bänderspannvorrichtung 1. Erkennbar sind dabei insbesondere die Skalen 31 und 32, 32', welche erfindungsgemäß wie bereits erwähnt die geplanten Resektionshöhen medial und lateral am Femur 34 unter Berücksichtigung der ligamentären Situation anzeigen und eine Messung der Resektionshöhen medial und lateral erlauben, um die korrekten Resektionshöhen senkrecht dazu, i.e. dorsal und ventral, zu bestimmen. Damit ist eine optimale Reproduktion der physiologischen Gelenksebene möglich, da sowohl die mediallaterale Richtung als auch die dorsal-ventrale Richtung in die Messung und damit in die Schnittführung der Resektion eingehen. Zusätzlich ist es durch die besondere Anordnung der Skalen 31, 32, 32' möglich, auch eine Rotationsbewegung des Femurs 34 zu kontrollieren, welche bei der Beugung und Streckung des Gelenks auftritt und bei nicht korrekter Einbeziehung zu Problemen im prothetisch versorgten Gelenk führen kann.

Die Skalen 31 und 32 sowie 31 und 32' korrespondieren dabei jeweils miteinander. Da die Bänderspannvorrichtung 1 mit zwei unabhängig voneinander arbeitenden Parallelverschiebevorrichtungen 12, 12' ausgestattet ist, welche unabhängig voneinander betätigbar sind, können medial und lateral somit unterschiedliche Weiten des Kniegelenkspalts bzw. des Inlays eingestellt werden, so daß die ligamentäre Situation des Gelenks optimal berücksichtigt werden kann.

Allgemein besteht eine Gelenkprothese aus mehreren Komponenten, welche je nach Zustand des Gelenks in einen oder in beide Knochenteile 33, 34 eingesetzt werden. Im Fall der prothetischen Versorgung des gesamten Gelenks ist eine Endoprothese nötig, welche zusätzlich ein Inlay umfassen kann, welches zwischen den Prothesenteilen liegt und im Fall des Kniegelenks die Funktion der Menisci übernimmt. Es ist zur zufriedenstellenden Versorgung des Patienten von Bedeutung, die Höhe des Inlays und vorbereitend die Resektionshöhe der beteiligten Knochen 33, 34 korrekt zu bestimmen.

Hierbei ist es sowohl hilfreich, die Höhe des Kniegelenksspalts in Flexion und Extension in diskreten Werten analog zu den verfügbaren Inlaygrößen einstellen zu können, als auch optional auf eine stufenlose Einstellung der Flexions- und Extensionsspalthöhe zurückgreifen zu können, welche kniespezifische Über-/Unterkorrekturen des Kniegelenkspalts durch eine stufenlose Knochenresektion erlaubt. Ferner ist es wünschenswert, die optimale ventrale Ausrichtung der Femurkomponente der Endoprothese sicher bestimmen zu können, welche den Übergang zwischen der Implantatkomponente relativ zur ventralen Kortikalis, also zum ventralen Auslauf, bestimmt.

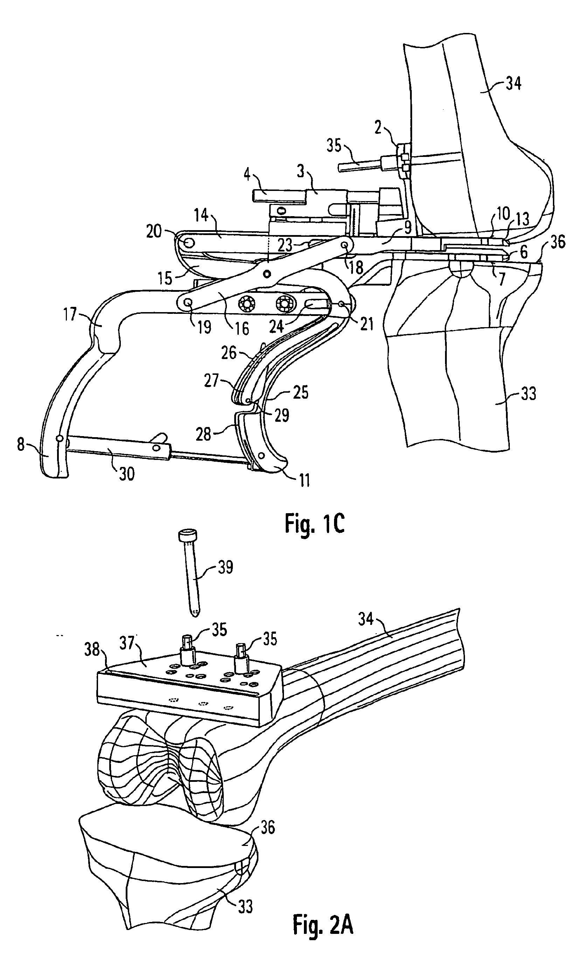

In Fig. 1C ist in einer seitlichen Ansicht die Situation gemäß Fig. 1A und 1B dargestellt. Die Bohrlehre 2 befindet sich dabei bereits in Anlage am Femur 34. Mittels eines Bohrers werden zwei Bohrungen in den Femur 34 eingebracht, welche, wie weiter unten beschrieben, eine Schnittlehre aufnehmen.

Anhand der folgenden Figuren und der dazugehörigen Beschreibung werden die zur korrekten Schnittführung benötigten vorbereitenden Schritte erläutert.

Wie nicht weiter dargestellt, wird die Tibia 33 durch herkömmliche Resektionsmethoden vorbereitet, so daß eine transversale Fläche 36 gebildet wird, an welcher die Pratzen 6, 6' der Bänderspannvorrichtung 1 anliegen.

Die Vorgehensweise zur Ermittlung der korrekten Inlaydicke bzw. der Resektionshöhe sowie die Vorbereitungen zur Resektion werden im folgenden erläutert.

Zunächst wird in Extension, also im gestreckten Zustand des Kniegelenks, die Bänderspannvorrichtung 1 in den Kniegelenkspalt zwischen Tibia 33 und Femur 34 eingeschoben. Die Bohrlehre 2 für die Einbringung der Bohrungen für die Schnittlehre, welche für eine erste, distale Femurresektion verwendet wird, ist bereits auf die Bänderspannvorrichtung 1 aufgesetzt.

Nun werden mittels Betätigung der Bänderspannvorrichtung 1 in Extensionsstellung die Ligamente unter einer wählbaren Kraft aufgespreizt. Die Kraft wird an den Skalen 26, 26' der Kraftanzeigen 25, 25' abgelesen und eingestellt. Die auf den Skalen 31, 32, 32' abgelesenen Werte ergeben die Resektionshöhe der ersten, distalen Femurresektion bzw. die Dicke des später einzulegenden Inlays zwischen den Prothesenkomponenten. Bedingt durch die getrennte Einstellbarkeit für die mediale und die laterale Seite können sich unterschiedliche Werte auf den Skalen 32, 32' ergeben, welche einer Rotation des Femur 34 entsprechen. Pro Millimeter der Skalen 32, 32' beträgt die Rotation vorzugsweise 1°.

Ist die an den Skalen 31, 32, 32' abzulesende Distanz zu groß, muß eine Nachresektion der Tibia 33 durchgeführt werden. Ist sie zu klein, muß eine größere Inlaygröße gewählt werden.

Nun werden mittels eines Bohrers zwei Bohrungen in den Femur 34 eingebracht, wie aus Fig. 1C ersichtlich. In die Bohrungen werden Stifte 35 eingesetzt. Danach wird die Bänderspannvorrichtung 1 entspannt und aus dem Kniegelenkspalt entfernt.

In den Fig. 2A bis 2C ist in verschiedenen Ansichten die Anbringung der für die distale Femurresektion benötigten Schnittlehre 37 dargestellt.

Das Gelenk wird, wie aus den Fig. 2A bis 2C ersichtlich, in Flexion gebeugt, also abgewinkelt, und die Schnittlehre 37 auf die Stifte 35 aufgesteckt. Damit sich die Schnittlehre 37 nicht verschieben kann, wird sie mittels eines Fixationsnagels 39 am Femur 34 fixiert. Die Schnittlehre 37 weist eine Sägeblattführung 38 auf, durch welche ein Sägeblatt bei der Resektion geführt wird.

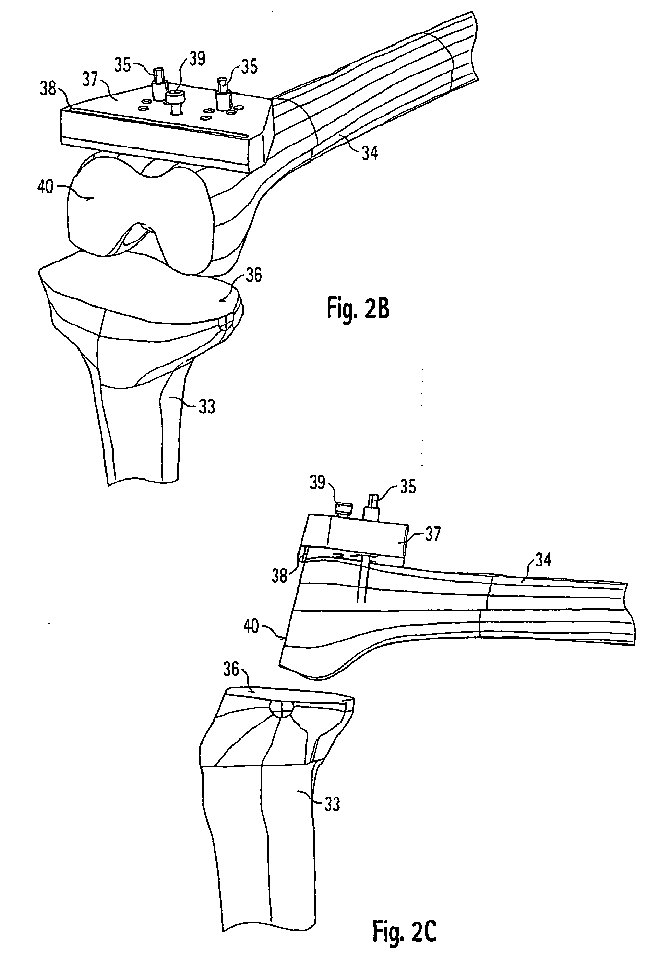

In Fig. 2B und 2C ist die Resektion bereits abgeschlossen, wodurch am Femur 34 ebenfalls eine transversal orientierte Fläche 40 gebildet worden ist, welche in Extension parallel zu der transversalen Fläche 36 der Tibia 33 orientiert ist.

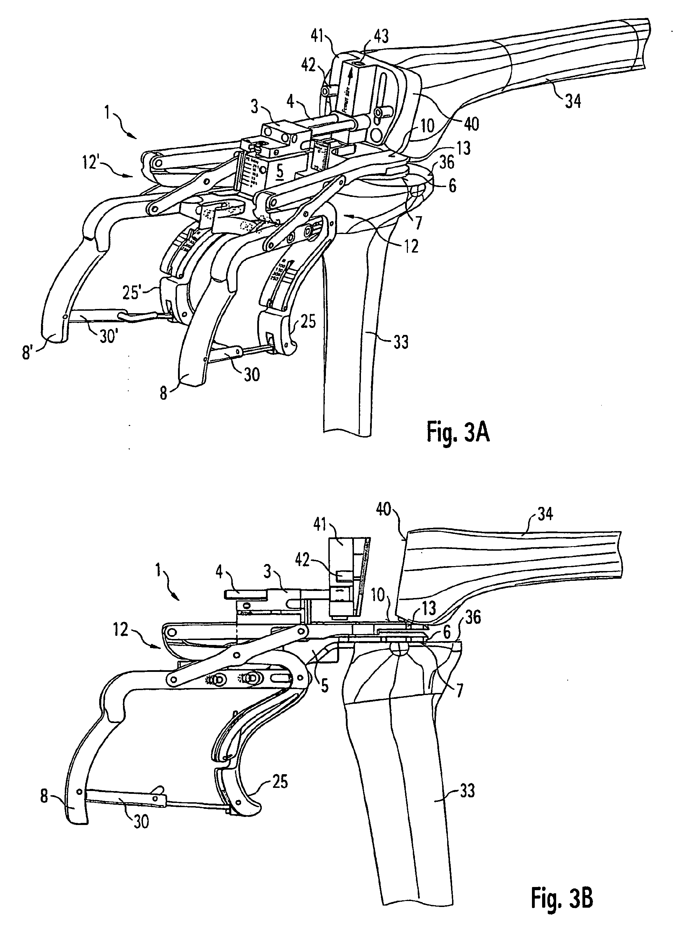

In den Fig. 3A bis 3D ist der nächste Schritt zur Vorbereitung der zweiten Femurresektion dargestellt.

Die Bänderspannvorrichtung 1 wird, wie in Fig. 3A in einer perspektivischen Gesamtdarstellung gezeigt, wieder an das immer noch in Flexion befindliche Gelenk angesetzt. Die Bohrlehre 2 ist durch eine zweite Bohrlehre 41 ersetzt, welche in gleicher Weise wie die Bohrlehre 2 an der Bänderspannvorrichtung 1 montiert wird.

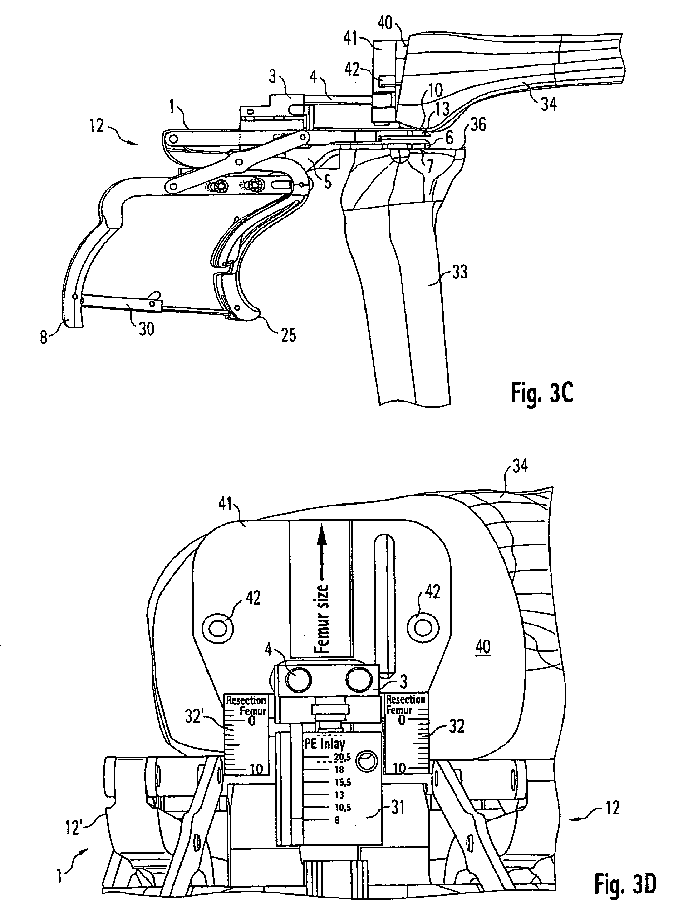

Fig. 3B und 3C zeigen in einer seitlichen Darstellung die Bohrlehre 41 im vormontierten Zustand bzw. nach der Anlage an die transversale Fläche 40 des Femurs 34. Die Flexion des Kniegelenks wird dabei so korrigiert, daß die Bohrlehre 41 und die Fläche 40 vollflächig in Anlage aneinander bringbar sind. Dies ist wichtig, um die korrekte Positionierung der Bohrungen zu gewährleisten.

Die Bohrlehre 41 weist zwei Führungen 42 für den Bohrer sowie eine Einschubbohrung 43 auf, in welche im nächsten Verfahrensschritt eine Tastlehre 44 zur Ermittlung der Femurgröße einschiebbar ist.

Fig. 3D zeigt die nochmalige Kontrolle der Resektionshöhe anhand der Skalen 31, 32, 32' vor der Ermittlung der Femurgröße mittels der Tastlehre 44, um sicherzustellen, daß später die korrekte Schnittlehre für die zweite Femurresektion gewählt wird.

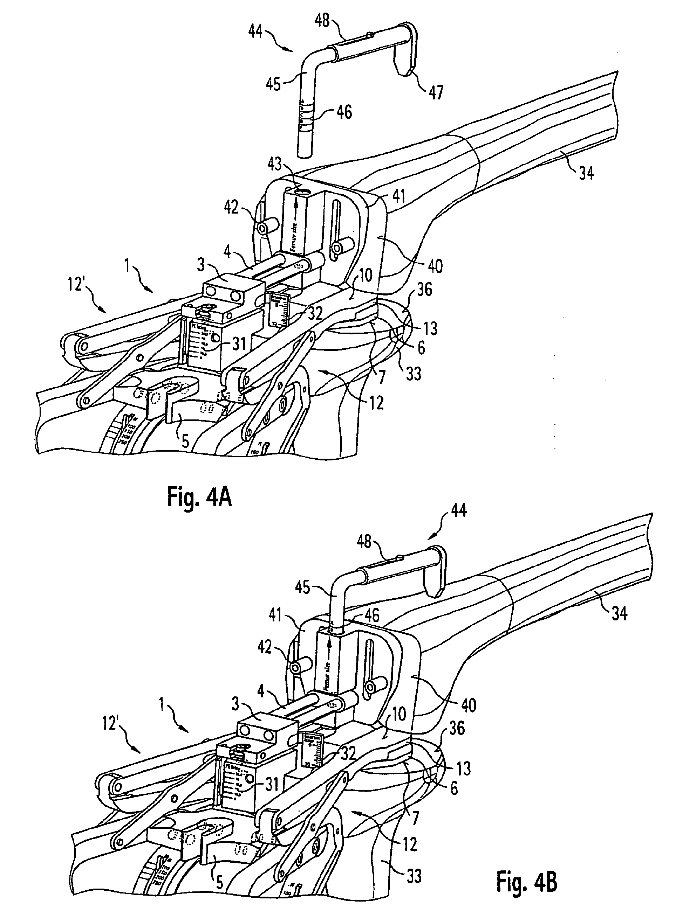

In den Fig. 4A bis 4C ist die Ermittlung der Femurgröße durch Abtasten mittels einer Tastlehre 44 dargestellt.

Die Tastlehre 44 weist einen L-förmigen Bügel 45 mit einer Skala 46 auf, welche an dem Teil des Bügels 45 ausgebildet ist, welcher in die Einschubbohrung 43 der Bohrlehre 41 eingeführt wird. Die Länge des Bügels 45 in proximaldistaler Richtung ist über eine Verschiebevorrichtung 48 veränderlich.

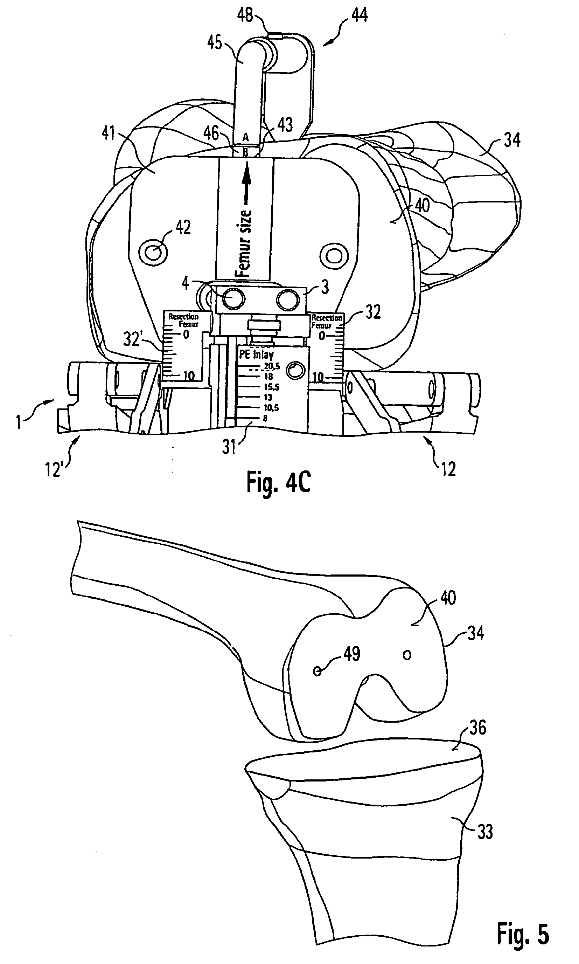

An einem der Skala 46 entgegengesetzten Ende der Tastlehre 44 ist ein Aufsatz 47 ausgebildet, welcher auf den Femur 34 aufgesetzt wird. Durch Einschieben der Tastlehre 44 in die Einschubbohrung 43 bis zum Aufsetzen des Aufsatzes 47 auf dem Femur 34 wird die Femurgröße ermittelt, welche an der Skala 46 abgelesen werden kann. Die Skala 46 weist im Ausführungsbeispiel fünf Markierungen A, B, C, D und E auf, welche fünf verschiedenen Femurgrößen entsprechen, wobei A die kleinste und E die größte Größe darstellt. Die Anzahl der Markierungen ist dabei nicht auf fünf begrenzt, sie kann auch mehr oder weniger betragen bzw. andere Abstände zwischen den Markierungen aufweisen. In dem in Fig. 4C dargestellten Ausführungsbeispiel ist die Femurgröße mit Markierung B ermittelt worden. Dies ist die Markierung, welche noch oberhalb der Einschubbohrung 43 in der Bohrlehre 44 sichtbar ist.

Nun werden durch die Führungen 42 zwei Bohrungen 49 in die Fläche 40 des Femur 34 eingebracht und anschließend die Bänderspannvorrichtung 1 entfernt. Fig. 5 zeigt die Situation nach dem Einbringen der Bohrungen 49 und nach dem Entfernen der Bänderspannvorrichtung 1 in Flexion. In die Fläche 40 sind die zwei Bohrungen 49 eingebracht, die transversal orientierten Flächen 36 und 40 sind in Extension des Kniegelenks dann parallel zueinander. Fig. 5 zeigt die Ausgangssituation für den letzten Bearbeitungsschritt der Vorbereitung auf die Implantatversorgung, nämlich für die ventralen und dorsalen Resektionen des Femurs 34.

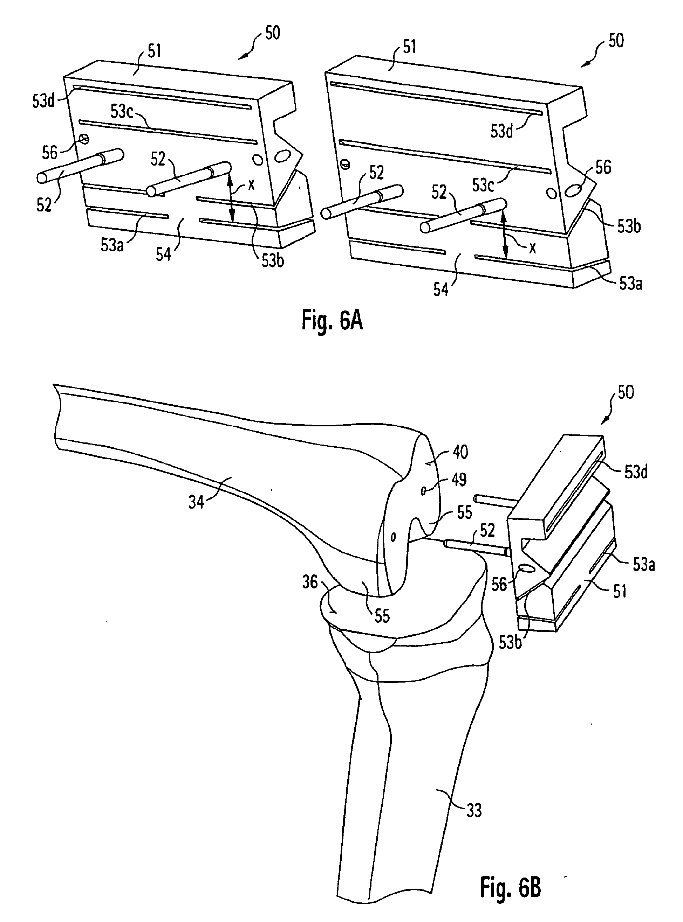

Zur Durchführung der verbleibenden Femurresektionen wird nur mehr eine einzige Schnittlehre 50 benötigt, welche beispielhaft in Fig. 6A dargestellt ist. Die Schnittlehre 50 ist dabei auf die vorher mit der Tastlehre 44 ermittelte Größe des Femur 34 abgestimmt, i.e. für jede der auf der Skala 46 angegebenen Femurgrößen A, B, C, D, E ist eine eigene Schnittlehre 50 vorgesehen, wobei sich die Schnittlehren 50 für die verschiedenen Femurgrößen in ihren Abmessungen unterscheiden.

In Fig. 6A sind beispielhaft die Schnittlehren 50 für einen kleinen Femur 34 der Größe A (links in Fig. 6A) und für einen großen Femur 34 der Größe E (rechts in Fig. 6A) dargestellt, um die Unterschiede zu verdeutlichen.

Die Schnittlehre 50 besteht dabei unabhängig von ihrer Dimensionierung aus einem Schnittblock 51, welcher zwei Stifte 52 zum Einführen in die vorher in die transversale Fläche 40 des Femur 34 eingebrachten Bohrungen 49 aufweist. Die Stifte 52 sind dabei ungefähr in Richtung einer Flächennormalen auf dem Schnittblock 51 angeordnet. Der Schnittblock 51 umfaßt weiterhin Sägeblattführungen 53, welche in unterschiedlichen Winkeln in dem Schnittblock 51 ausgebildet sind. Die Anzahl der Sägeblattführungen 53 beträgt dabei vier, welche für jeweils einen dorsalen Femurschnitt, einen dorsalen Schrägschnitt, einen ventralen Schrägschnitt und einen ventralen Femurschnitt angelegt sind.

Der Schnittblock 51 ist dabei so ausgelegt, daß ein Abstand X zwischen einer ersten Sägeblattführung 53a für einen dorsalen Femurschnitt, welche in Fig. 6A bis 6D jeweils die unterste (dorsale) Sägeblattführung 53 ist, und den Stiften 52 für alle Femurgrößen A, B, C, D, E gleich groß ist. Dies hat den Vorteil, daß der dorsale Femurschnitt immer an der gleichen Stelle erfolgt und somit später die Endoprothese relativ zum Femur 34 stets richtig positioniert werden kann.

Die Sägeblattführung 53a für den dorsalen Femurschnitt ist dabei zweiteilig ausgeführt, wobei die beiden Teilschlitze durch einen Steg 54 voneinander getrennt sind. Dies ist zweckmäßig, um die Stabilität des Schnittblocks 51 zu erhöhen. Die nächstfolgende Sägeblattführung 53b ist für einen dorsalen Schrägschnitt geneigt zu der ersten Sägeblattführung 53a angeordnet. Die Sägeblattführung 53b für den dorsalen Schrägschnitt ist ebenfalls zweiteilig ausgebildet und durch den Steg 54 geteilt.

Eine weitere Sägeblattführung 53c für einen ventralen Schrägschnitt ist in Form eines rundum geschlossenen Schlitzes ausgeführt und gegenüber der für den dorsalen Schrägschnitt zu verwendenden Sägeblattführung 53b um etwa 90° geneigt. Eine vierte Sägeblattführung 53d ist ebenfalls rundum geschlossen und zur Durchführung des abschließenden ventralen Femurschnitts zu verwenden.

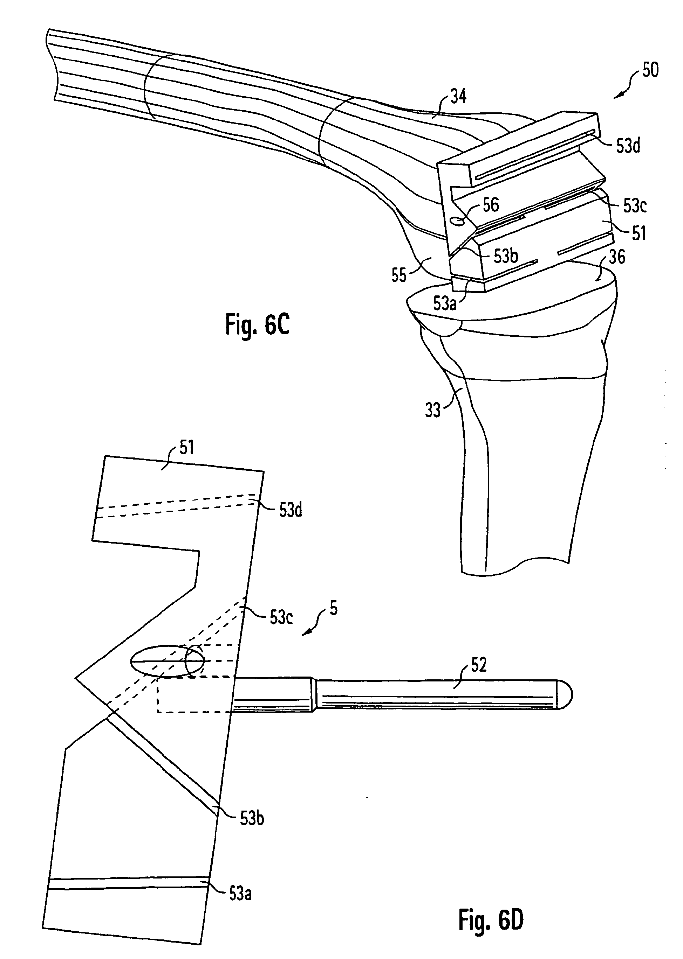

Die zweiteiligen Sägeblattführungen 53a, 53b sind dabei von ihrer Dimensionierung so auszulegen, daß die Kondylen 55 des Femur 34 zuverlässig rezessiert werden können. Der Steg 54 muß also schmal genug sein, um die vollständige Rezession zu ermöglichen. Fig. 6D zeigt in einer seitlichen Ansicht eine Schnittlehre 50, bei welcher die beiden Sägeblattführungen 53a und 53b seitlich einsehbar sind, während die beiden ventralen Sägeblattführungen 53c und 53d in Fig. 6D nur in der Projektion sichtbar sind, da sie als rundum geschlossene Schlitze ausgeführt sind.

Weiterhin sind in dem Schnittblock 51 im Beispiel zwei Bohrungen 56 ausgebildet, welche zur Fixierung des Schnittblocks 51 am Femur 34 dienen. Durch die Bohrungen 56 können Fixationsnägel in die transversale Fläche 40 des Femur 34 eingeschlagen werden. Dadurch wird sichergestellt, daß sich die Schnittlehre 50 während der vier Resektionsschnitte nicht verschiebt.

Fig. 6B und 6C zeigen das Ansetzen der Schnittlehre 50 an das immer noch in Flexion befindliche Kniegelenk bzw. die Schnittlehre 50 in situ.

Dadurch, daß die Schnittlehre nur einmal positioniert werden muß und dann für alle vier benötigten Femurresektionen am Platz bleiben kann, wird einerseits die Handhabung der Schnittlehre 50 für den Operateur erheblich vereinfacht. Andererseits ist die Bearbeitungsgenauigkeit höher, so daß Nachresektionen entfallen können, und die Operationsdauer kann erheblich verkürzt werden, da die Schnittlehre 50 nicht vor jeder Resektion aufs Neue positioniert werden muß. Dies ist insbesondere in Hinblick auf die Verwendung von Navigationssystemen mit elektronischer Steuerung von Vorteil, da der Kalibrierungsprozeß des Navigationssystems aufwendig ist und bei der Schnittlehre 50 nur einmal erfolgen muß.

Die Erfindung ist nicht auf das dargestellte Ausführungsbeispiel beschränkt, sondern auch für Bänderspannvorrichtungen 1 für andere kugelige Gelenke bei entsprechender Anpassung anwendbar.

## Patentansprüche

1. Bänderspannvorrichtung (1) zur Vorbereitung für die Implantierung eines Gelenkimplantats mit einem Grundkörper (5), welcher erste Pratzen (6, 6') mit ersten medialen und lateralen Anlageflächen (7, 7') aufweist, welche auf einem ersten Knochenteil (33) aufliegen, und zweite Pratzen (13, 13'), die mit zweiten medialen und lateralen Anlageflächen (10, 10') an einem zweiten Knochenteil (34) aufliegen, wobei jeweils die medialen und lateralen Pratzen (6, 6', 13, 13') durch eine mediale und eine laterale Parallelverschiebevorrichtung (12, 12') zueinander verschieblich sind, und mit einer ersten Skala (31), die an dem Grundkörper (5) der Bänderspannvorrichtung (1) angeordnet ist, wobei zweite mediale und laterale Skalen (32, 32') vorgesehen sind, wobei mithilfe der medialen Skala (32) eine geplante mediale Resektionshöhe gemessen werden kann und mithilfe der lateralen Skala (32') eine geplante laterale Resektionshöhe,
**dadurch gekennzeichnet,**
**dass** die zweiten Skalen (32, 32') an einem Führungskörper (3) angeordnet sind, welcher gegenüber dem Grundkörper (5) verschiebbar gelagert ist, wobei durch Verschiebung des Führungskörpers (3) mittels der ersten Skala (31) die Höhe eines in das Gelenk einzusetzenden Implantats voreinstellbar ist.

2. Bänderspannvorrichtung nach Anspruch 1,
**dadurch gekennzeichnet,**
**dass** die zweiten Skalen (32, 32') medialer- und lateralerseits des Führungskörpers (3) angeordnet sind.

3. Bänderspannvorrichtung nach Anspruch 1 oder 2,
**dadurch gekennzeichnet,**
**dass** in dem Führungskörper (3) Führungsstäbe (4) ausgebildet sind.

4. Bänderspannvorrichtung nach Anspruch 3,
**dadurch gekennzeichnet,**
**dass** die Führungsstäbe (4) ventral-dorsal orientiert sind.

5. Bänderspannvorrichtung nach Anspruch 3 oder 4,
**dadurch gekennzeichnet,**
**dass** die Führungsstäbe (4) in ventral-dorsaler Richtung in dem Führungskörper (3) verschieblich sind.

6. Bänderspannvorrichtung nach einem der Ansprüche 3 bis 5,
**dadurch gekennzeichnet,**
**dass** auf die Führungsstäbe (4) Bohrlehren (2, 41) aufsteckbar sind.

7. Bänderspannvorrichtung nach Anspruch 6,
**dadurch gekennzeichnet,**
**dass** eine Position der Bohrlehren (2, 41) relativ zu dem zweiten Knochenteil (34) über die Bänderspannvorrichtung (1) und die Führungsstäbe (4) in zwei Freiheitsgraden veränderlich ist.

## Claims

1. A ligament-tensioning device (1) for preparation for implantation of an articulated implant having a base body (5) which has first claws (6, 6') with first medial and lateral bearing surfaces (7, 7'), resting on a first bone portion (33), and second claws (13, 13'), resting with second medial and lateral bearing surfaces (10, 10') on a second bone portion (34), wherein in each case the medial and lateral claws (6, 6', 13, 13') can be displaced in relation to each other by means of a medial and a lateral parallel displacement device (12, 12'), and having a first scale (31) which is arranged on the base body (5) of the ligament-tensioning device (1), wherein second medial and lateral scales (32, 32') are provided, wherein it is possible to measure a planned medial resection height with the aid of the medial scale (32) and a planned lateral resection height with the aid of the lateral scale (32'),
**characterised in that**
the second scales (32, 32') are arranged on a guide body (3) which is mounted so as to be displaceable with respect to the base body (5), wherein by displacement of the guide body (3) by means of the first scale (31) it is possible to pre-adjust the height of an implant that is to be inserted into the joint.

2. A ligament-tensioning device according to claim 1,
**characterised in that**
the second scales (32, 32') are arranged on the medial and lateral sides of the guide body (3).

3. A ligament-tensioning device according to claim 1 or 2,
**characterised in that**
guide rods (4) are formed in the guide body (3).

4. A ligament-tensioning device according to claim 3,
**characterised in that**
the guide rods (4) are oriented in a ventral-dorsal direction.

5. A ligament-tensioning device according to claim 3 or 4,
**characterised in that**
the guide rods (4) are displaceable in the ventral-dorsal direction in the guide body (3).

6. A ligament-tensioning device according to one of claims 3 to 5,
**characterised in that**
drilling jigs (2, 41) can be plugged onto the guide rods (4).

7. A ligament-tensioning device according to claim 6,
**characterised in that**
it is possible to vary a position of the drilling jigs (2, 41) relative to the second bone portion (34) by way of the ligament-tensioning device (1) and the guide rods (4) in two degrees of freedom.

## Revendications

1. Dispositif de tension de ligaments (1) pour la préparation à l'implantation d'un implant articulaire doté d'un corps de base (5) qui présente des premières pattes (6, 6') comportant des premières surfaces d'appui médiale et latérale (7, 7') qui reposent sur une première partie osseuse (33) et des deuxièmes pattes (13, 13') comportant des deuxièmes surfaces d'appui médiale et latérale (10, 10') sur une deuxième partie osseuse (34), les pattes médiale et latérale respectives (6, 6', 13, 13') pouvant être décalées les unes par rapport aux autres par un dispositif de décalage parallèle médial et latéral (12, 12') et comportant une première échelle (31) qui est disposée sur le corps de base (5) du dispositif de tension de ligaments (1), des deuxièmes échelles médiale et latérale (32, 32') étant prévues, dans lequel à l'aide de l'échelle médiale (32), une hauteur de résection médiale prévue peut être mesurée et à l'aide de l'échelle latérale (32'), une hauteur de résection latérale prévue peut être mesurée,
**caractérisé en ce que**
les deuxièmes échelles (32, 32') sont disposées sur un corps de guidage (3) qui est placé de façon à pouvoir coulisser de l'autre côté du corps de base (5), la hauteur d'un implant à insérer dans l'articulation pouvant être prédéfinie par décalage du corps de guidage (3) au moyen de la première échelle (31).

2. Dispositif de tension de ligaments selon la revendication 1,
**caractérisé en ce que**
les deuxièmes échelles (32, 32') sont disposées sur les côtés médial et latéral du corps de guidage (3).

3. Dispositif de tension de ligaments selon la revendication 1 ou 2,
**caractérisé en ce que**
dans le corps de guidage (3) sont formées des tiges de guidage (4).

4. Dispositif de tension de ligaments selon la revendication 3,
**caractérisé en ce que**
les tiges de guidage (4) ont une orientation ventrale - dorsale.

5. Dispositif de tension de ligaments selon la revendication 3 ou 4,
**caractérisé en ce que**
les tiges de guidage (4) peuvent être décalées dans une direction ventrale - dorsale dans le corps de guidage (3).

6. Dispositif de tension de ligaments selon l'une des revendications 3 à 5,
**caractérisé en ce que**
sur les tiges de guidage (4), des gabarits d'alésage (2, 41) peuvent être placés.

7. Dispositif de tension de ligaments selon la revendication 6,
**caractérisé en ce que**
une position des gabarits d'alésage (2, 41) peut varier en deux degrés de liberté par rapport à la deuxième partie osseuse (34) par le biais du dispositif de tension de ligaments (1) et des tiges de guidage.
